# EUROPÄISCHE PATENTSCHRIFT

(11) **EP 1 547 535 B1**
(45) Veröffentlichungstag und Bekanntmachung des Hinweises auf die Patenterteilung: **14.10.2009**
(21) Anmeldenummer: 04024941.9
(22) Anmeldetag: 20.10.2004
(51) Int. Cl.: A61B 17/86

(54) **Selbstbohrende Knochenschraube**
Self-drilling bone screw
Vis d'osteosynthèse auto-forante

(30) Priorität: 23.12.2003 DE 10361044
(43) Veröffentlichungstag der Anmeldung: 29.06.2005
(73) Patentinhaber: Stryker Leibinger GmbH & Co. KG, 79111 Freiburg (DE)
(72) Erfinder: Auth, Stefan, 79291 Merdingen (DE)
(74) Vertreter: Röthinger, Rainer

(56) Entgegenhaltungen:
- EP-A- 1 175 181
- WO-A-00/40165
- US-A- 4 903 691
- US-A1- 2001 004 694
- US-B1- 6 221 075

## Beschreibung

### GEBIET DER ERFINDUNG

Die Erfindung betrifft eine selbstbohrende Knochenschraube, insbesondere zur Verwendung bei chirurgischen Eingriffen im cranio-facialen Bereich, die einen mit einem Gewinde versehenen Schaft aufweist.

### HINTERGRUND DER ERFINDUNG

Bei chirurgischen Eingriffen im cranio-facialen Bereich ist es häufig erforderlich, ein aus der Schädelkalotte ausgesägtes Schädelfragment nach Beendigung des chirurgischen Eingriffs zu repositionieren und anschließend zu fixieren oder, alternativ dazu, operativ entfernte Knochenbereiche durch eine aus einem geeigneten biokompatiblen Material, wie z.B. Titan bestehende Platte zu substituieren. Da im cranio-facialen Bereich keine großen Kräfte auf die entsprechenden Fixierungsstellen wirken, spielt in diesem anatomischen Bereich die Festigkeit und insbesondere die Ausreißfestigkeit der Fixierung nur eine untergeordnete Rolle. Zur Befestigung des ausgesägten Schädelfragments oder der Platte in der gewünschten Position werden daher bevorzugt selbstbohrende Knochenschrauben eingesetzt. Derartige selbstbohrende Knochenschrauben weisen den Vorteil auf, dass sie vom Operateur auf verhältnismäßig einfache Art und Weise in einen Knochen oder ein Knochenfragment eingeschraubt werden können, ohne dass eine vorbereitende Behandlung des Knochens oder des Knochenfragments, beispielsweise durch das Einbringen einer Bohrung oder dergleichen erforderlich ist.

Um ein einfaches Einschrauben in den Knochen oder das Knochenfragment zu ermöglichen, weisen selbstbohrende Knochenschrauben üblicherweise eine Schraubenspitze mit einem möglichst kleinen Spitzenwinkel auf. Insbesondere bei längeren Schrauben tritt dann jedoch häufig das Problem auf, dass sich die Schraubenspitze beim Einschrauben in den Knochen oder das Knochenfragment verbiegt oder gar bricht.

Aus der US 5,925,048 ist eine selbstbohrende Knochenschraube bekannt, die einen Kopf sowie einen mit einem Gewinde versehenen Schaft umfasst. Die Schraube weist eine Gesamtlänge von 4 bis 14 mm und einen Spitzenwinkel von 45 bis 50° auf.

Die US 4,903,691 beschreibt eine selbstbohrende Knochenschraube, die ein Gewinde mit einer Gewindesteigung von ungefähr 1 mm umfasst.

Aus dem Artikel mit dem Titel "In vitro evaluation of self-drilling maxillofacial screws. A histomorphological and functional investigation", J. Löhr et al., Mund Kiefer GesichtsChir (2000), 4: 159-163 ist eine selbstbohrende verdrängende Knochenschraube mit einer Gewindesteigung von 0,75 mm bekannt.

Der Erfindung liegt die Aufgabe zugrunde, eine selbstbohrende Knochenschraube zur Verfügung zu stellen, die auf einfache und rasche Art und Weise in einen Knochen oder ein Knochenfragment eingeschraubt werden kann und bei der gleichzeitig ein Verbiegen oder gar Brechen der Schraubenspitze beim Einschrauben zuverlässig vermieden wird.

### ZUSAMMENFASSUNG DER ERFINDUNG

Erfindungsgemäß wird zur Lösung dieser Aufgabe eine selbstbohrende Knochenschraube mit einem mit einem Gewinde versehenen Schaft vorgeschlagen, bei der das Gewinde eine Gewindesteigung von 0,8 bis 0,9 mm aufweist. Die Knochenschraube weist einen flankenlos ausgebildeten Spitzenbereich mit einem Spitzenwinkel auf, der so gewählt ist, dass der Beginn einer Gewindeflanke eines sich an den Spitzenbereich der Knochenschraube anschließenden ersten Gewindegangs 0,2 bis 0,5 mm von einer Spitze der Knochenschraube entfernt angeordnet ist. Das Gewinde kann als Linksgewinde oder als Rechtsgewinde ausgebildet sein.

Bei einer Gewindesteigung von 0,8-0,9 mm liegt der Spitzenwinkel in einem Bereich von 34 bis 43°. In Abhängigkeit der gewählten Gewindesteigung kann auch ein in einem Bereich von ungefähr 35 bis 38° liegender Spitzenwinkel vorteilhaft sein.

Eine in diesem Bereich liegende, verhältnismäßig große Gewindesteigung erlaubt ein schnelles Eindrehen der Schraube in einen Knochen oder ein Knochenfragment. Schrauben, bei denen die Gewindesteigung ungefähr 0,8 mm bzw. ungefähr 0,9 mm und der Spitzenwinkel ungefähr 43° bzw. ungefähr 36° beträgt, weisen ein besonders gutes Anschneideverhalten auf.

Die erfindungsgemäße selbstbohrende Knochenschraube weist typischerweise eine Länge von ungefähr 3 bis 7 mm auf. Bei einer Ausführungsform der Erfindung beträgt die Länge der Schraube ungefähr 3,5 bis 5 mm. Eine Knochenschraube dieser Länge ist für den Einsatz im cranio-facialen Bereich sehr gut geeignet.

Ein Durchmesser eines Gewindekerns der erfindungsgemäßen Knochenschraube kann in einem Bereich von ungefähr 1,0 bis 1,3 mm liegen. Vorteilhaft ist ein Gewindekerndurchmesser von ungefähr 1,05 bis 1,2 mm. Ein derartiger, verhältnismäßig groß gewählter Gewindekerndurchmesser gewährleistet, dass die Schraube beim Eindrehen in den Knochen oder das Knochenfragment nicht bricht. Bei einer Schraubenlänge von 4,5 mm hat sich ein Gewindekerndurchmesser von 1,1 mm als besonders vorteilhaft erwiesen.

Bei einer weiteren Ausführungsform der erfindungsgemäßen selbstbohrenden Knochenschraube ist der Gewindekern konisch ausgebildet. Dadurch wird das erforderliche Drehmoment zum Eindrehen der ersten Gewindegänge verringert, so dass die Schraube besonders einfach in den Knochen oder das Knochenfragment eingeschraubt werden kann. Der Gewindekern kann auch zylindrisch ausgebildet sein.

Vorzugsweise umfasst die Knochenschraube einen Kopf. Im Bereich des Kopfes kann eine Drehmomentaufnahmestruktur wie ein Schlitz oder ein Kreuzschlitz ausgebildet sein. Auch Hexagonal- oder Torxstrukturen könnten Verwendung finden. Die Drehmomentaufnahmestruktur weist vorzugsweise eckig ausgebildete Kanten auf. Die Knochenschraube kann dann unter Verwendung eines bei chirurgischen Eingriffen eingesetzten, konventionellen Schraubendrehers eingeschraubt werden. Die eckig ausgebildeten Kanten verhindern, dass der Schraubendreher insbesondere dann, wenn der Schraubendreher abgewinkelt werden muss, aus der Drehmomentstruktur herausrutscht.

Bei einer Ausführungsform der erfindungsgemäßen Knochenschraube weist der Kopf der Knochenschraube einen Durchmesser von ungefähr 2,0 bis 3,0 mm auf. Besonders vorteilhaft ist ein Schraubenkopfdurchmesser von ungefähr 2,55 bis 2,65 mm. Dieser verhältnismäßig große Schraubenkopfdurchmesser ermöglicht es dem Operateur, über die Schraubendreherklinge ein großes Drehmoment auf die Schraube zu übertragen. Dadurch wird das Eindrehen der Schraube in den Knochen oder das Knochenfragment erleichtert. Der Schraubenkopf könnte jedoch auch weggelassen werden. In einem solchen Fall würde die Schraube nur aus Gewinde und Schaft bestehen. Der Schaft kann eine Drehmomentaufnahmestruktur besitzen.

Die Knochenschraube ist vorzugsweise dazu eingerichtet, eine in einer Platte ausgebildete Bohrung zu durchsetzen. Die Platte kann beispielsweise eine Halteplatte zur Verbindung zweier oder mehrerer Knochen oder Knochenfragmente sein, die mit Hilfe der erfindungsgemäßen Knochenschraube an die Knochenfragmente angeschraubt werden kann. Alternativ dazu kann die Platte auch dazu vorgesehen sein, ein operativ entferntes Knochenfragment zu ersetzen und mit Hilfe der erfindungsgemäßen Knochenschraube an zu dem entfernten Knochenfragment benachbarten Knochenbereichen fixiert zu werden. Eine Unterseite des Kopfes der Knochenschraube kann an eine in einer Oberfläche der Platte ausgebildeten Senkung angepasst sein. Durch das Einsenken des Kopfes der erfindungsgemäßen Knochenschraube in die in die Oberfläche der Platte eingeformte Senkung weisen die Elemente Platte/Schraubenkopf eine vergleichsweise geringe Höhe auf. Die Ausgestaltung dieser Elemente mit einer vergleichsweise geringen Höhe von vorzugsweise 0,6 bis 0,7 mm hat den Vorteil, dass ein am Cranium operierter Patient die in einem anatomischen Bereich implantierte Platte, in dem sich nur wenig Weichteilgewebe befindet, unter der Haut nicht spürt.

Bei einer besonders einfach und kostengünstig zu fertigenden Ausführungsform der erfindungsgemäßen selbstbohrenden Knochenschraube ist die Unterseite des Kopfes der Knochenschraube konisch ausgebildet. Der Schraubenkopf kann dann in eine ebenfalls einfach und kostengünstig herzustellende konisch geformte Senkung in der Oberfläche der Platte eingesenkt werden.

### KURZE BESCHREIBUNG DER ZEICHNUNGEN

Weitere Vorteile und Ausgestaltungen der Erfindung ergeben sich aus der nachfolgenden Beschreibung bevorzugter Ausführungsbeispiele und den Figuren. Es zeigen:
- Fig. 1: eine Querschnittsansicht einer erfindungsgemäßen selbstbohrenden Knochenschraube;
- Fig. 2: eine perspektivische Ansicht der erfindungsgemäßen selbstbohrenden Knochenschraube;
- Fig. 3: eine teilweise geschnittene Darstellung, in der die erfindungsgemäße selbstbohrende Knochenschraube eine in einer Platte ausgebildete Bohrung durchsetzt;

### BESCHREIBUNG BEVORZUGTER AUSFÜHRUNGSBEISPIELE

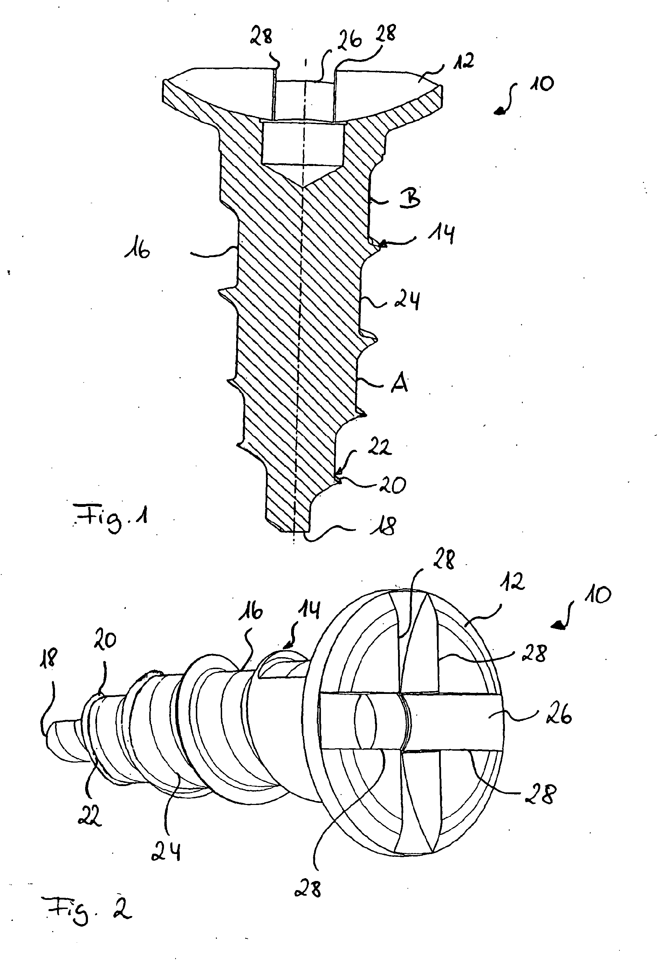

Eine in den Fig. 1 bis 3 gezeigte selbstbohrende Knochenschraube 10 umfasst einen Kopf 12 sowie einen mit einem Gewinde 14 versehenen Schaft 16. In dem gezeigten Ausführungsbeispiel hat die zur Verwendung bei chirurgischen Eingriffen im cranio-facialen Bereich vorgesehene Schraube 10 eine Gesamtlänge von ungefähr 5 mm. Es versteht sich jedoch, dass in Abhängigkeit der gewünschten Anwendung auch andere Schraubenlängen, beispielsweise 4 mm gewählt werden können. Um ein rasches Eindrehen der Schraube 10 in einen Knochen oder ein Knochenfragment zu ermöglichen, weist das Gewinde 14 eine Gewindesteigung von ungefähr 0,9 mm auf.

Ein Spitzenwinkel einer Schraubenspitze 18 ist so gewählt, dass der Beginn einer Gewindeflanke 20 eines sich an die Schraubenspitze 18 anschließenden ersten Gewindegangs 22 in einer Entfernung von ungefähr 0,32 mm von der Schraubenspitze 18 angeordnet ist, wobei der vorderste Spitzenbereich flankenlos ausgebildet ist. Um dies zu gewährleisten, beträgt der Spitzenwinkel in dem gezeigten Ausführungsbeispiel der Knochenschraube 10 ungefähr 36°. Durch die Anpassung des Spitzenwinkels an die Gewindesteigung wird sichergestellt, dass die Schraubenspitze 18 so dimensioniert ist, dass ein Verbiegen oder gar Brechen der Schraubenspitze 18 beim Eindrehen der Schraube 10 in einen Knochen oder ein Knochenfragment zuverlässig vermieden wird. Gleichzeitig sorgt die in einem Abstand von ungefähr 0,32 mm vom der Schraubenspitze 18 angeordnete erste Gewindeflanke 20 dafür, dass die Schraube 10 beim Einschrauben in den Knochen "gezogen" wird, wodurch ein gutes Anschneideverhalten der Schraube 10 gewährleistet wird.

Bei einem weiteren, in den Figuren nicht gezeigten Ausführungsbeispiel beträgt die Gewindesteigung ungefähr 0,8 mm, wobei die Gewindeflanke des sich an die Schraubenspitze anschließenden ersten Gewindegangs ebenfalls in einer Entfernung von ungefähr 0,32 mm von der Schraubenspitze angeordnet ist. Der Spitzenwinkel dieser Schraube beträgt ungefähr 43°.

Um das zum Eindrehen der ersten Gewindegänge des Gewindes 14 in den Knochen oder das Knochenfragment erforderliche Drehmoment zu verringern, ist ein Gewindekern 24 der Schraube 10 konisch ausgebildet. In der in Fig. 1 mit A bezeichneten Position hat der Gewindekern einen Durchmesser von ungefähr 1,1 mm. An der in Fig. 1 mit B bezeichneten Position beträgt der Gewindekerndurchmesser ungefähr 1,15 mm. Dieser verhältnismäßig große Gewindekerndurchmesser gewährleistet, dass die Schraube beim Eindrehen in den Knochen oder das Knochenfragment nicht bricht.

Am Kopf 12 der Knochenschraube 10 ist ein Kreuzschlitz 26 zu Aufnahme einer Klinge eines bei chirurgischen Eingriffen üblicherweise eingesetzten konventionellen Schraubendrehers vorgesehen. Um insbesondere dann, wenn der Schraubendreher abgewinkelt werden muss, ein Herausrutschen der Klinge aus dem Kreuzschlitz 26 zu vermeiden, ist der Kreuzschlitz mit eckig geformten Kanten 28 ausgebildet.

Der Kopf 12 der Knochenschraube 10 weist einen Durchmesser von ungefähr 2,6 mm auf. Dieser verhältnismäßig große Schraubenkopfdurchmesser ermöglicht es dem Operateur, über die Schraubendreherklinge ein großes Drehmoment auf die Schraube 10 zu übertragen. Dadurch wird das Eindrehen der Schraube 10 in den Knochen oder das Knochenfragment erleichtert.

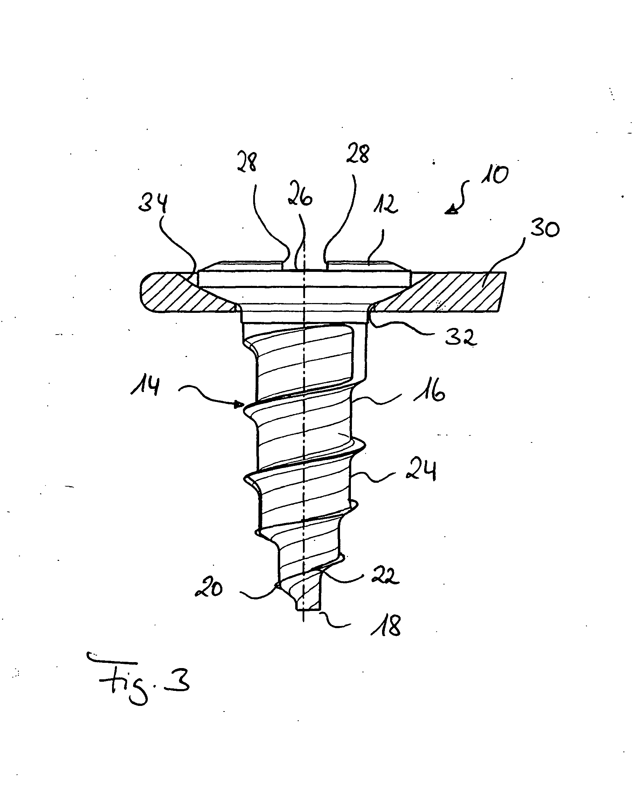

Wie in Fig. 3 gezeigt ist, ist die Knochenschraube 10 im implantierten Zustand dazu vorgesehen, eine in einer Platte 30 ausgebildete Bohrung 32 zu durchsetzen. Die Platte 30 kann beispielsweise eine Halteplatte zur Verbindung zweier oder mehrerer Knochenfragmente sein, die mit Hilfe der Knochenschraube 10 an die Knochenfragmente angeschraubt wird. Alternativ dazu kann die Platte 30 auch dazu vorgesehen sein, ein operativ entferntes Knochenfragment zu ersetzen und mit Hilfe der Knochenschraube 10 an zu dem entfernten Knochenfragment benachbarten Knochenbereichen fixiert zu werden.

Eine Unterseite des Kopfes 12 der Knochenschraube 10 ist, wie am besten in den Fig. 1 und 3 zu erkennen ist, konisch ausgebildet. Dadurch kann der Schraubenkopf 12 in eine in einer Oberfläche der Platte 30 ausgebildet ebenfalls konisch geformte Senkung 34 eingesenkt werden. Durch das Einsenken des Schraubenkopfes 12 in die in die Oberfläche der Platte eingeformte Senkung weisen die Elemente Platte/Schraubenkopf eine vergleichsweise geringe Höhe von ungefähr 0,65 mm auf. Dadurch wird sichergestellt, dass ein am Cranium operierter Patient die in einem anatomischen Bereich implantierte Platte, in dem sich nur wenig Weichteilgewebe befindet, unter der Haut nicht spürt.

Der Anwendungsbereich der vorstehend erläuterten Knochenschraube 10 ist nicht auf das cranio-facialen Gebiet beschränkt. Die Knochenschraube 10 könnte beispielsweise auch im Handbereich eingesetzt werden.

## Patentansprüche

1. Selbstbohrende Knochenschraube (10), die einen mit einem Gewinde (14) versehenen Schaft (16) sowie einen flankenlos ausgebildeten Spitzenbereich aufweist, **dadurch gekennzeichnet, dass** das Gewinde (14) eine Gewindesteigung von 0,8 bis 0,9 mm aufweist, die Knochenschraube (10) einen Spitzenwinkel von 34 bis 43° besitzt und der Beginn einer Gewindeflanke (20) eines sich an den Spitzenbereich der Knochenschraube (10) anschließenden ersten Gewindegangs (22) 0,2 bis 0,5 mm von einer Spitze (18) der Knochenschraube (10) entfernt angeordnet ist.

2. Selbstbohrende Knochenschraube nach Anspruch 1, **dadurch gekennzeichnet, dass** die Knochenschraube (10) eine Länge von 3 bis 7 mm aufweist.

3. Selbstbohrende Knochenschraube nach einem der Ansprüche 1 bis 2, **dadurch gekennzeichnet, dass** ein Gewindekern (24) einen Durchmesser von 1,0 bis 1,3 mm aufweist.

4. Selbstbohrende Knochenschraube nach Anspruch 3, **dadurch gekennzeichnet, dass** der Gewindekern (24) einen Durchmesser von 1,05 bis 1,2 mm aufweist.

5. Selbstbohrende Knochenschraube nach einem der Ansprüche 1 bis 4, **dadurch gekennzeichnet, dass** der Gewindekern (24) konisch ausgebildet ist.

6. Selbstbohrende Knochenschraube nach einem der Ansprüche 1 bis 5, **dadurch gekennzeichnet, dass** die Knochenschraube (10) einen Kopf (12) mit einer Drehmomentaufnahmestruktur (26) umfasst, wobei die Drehmomentaufnahmestruktur (26) eckig ausgebildete Kanten (28) aufweist.

7. Selbstbohrende Knochenschraube nach Anspruch 6, **dadurch gekennzeichnet, dass** der Kopf (12) der Knochenschraube (10) einen Durchmesser von 2,0 bis 3,0 mm aufweist.

8. Implantatsystem mit einer selbstbohrenden Knochenschraube (10) nach einem der Ansprüche 1 bis 7 und einer Platte (30), wobei die Knochenschraube (10) dazu eingerichtet ist, eine in der Platte (30) ausgebildete Bohrung (32) zu durchsetzen, wobei eine Unterseite eines Kopfes (12) der Knochenschraube (10) an eine in einer Oberfläche der Platte (30) ausgebildete Senkung (34) angepasst ist.

9. Implantatsystem nach Anspruch 8, **dadurch gekennzeichnet, dass** die Unterseite des Kopfes (12) der Knochenschraube (10) konisch ausgebildet ist.

## Claims

1. A self-drilling bone screw (10) comprising a shank (16) provided with a thread (14) and a tip region constructed without a flank, **characterized in that** the thread (14) has a pitch of 0.8 to 0.9 mm, the bone screw (10) has a tip angle of 34 to 43° and the start of a thread flank (20) of a first flight (22) adjacent to a tip region of the bone screw (10) is arranged at a distance of 0.2 to 0.5 mm from a tip (18) of the bone screw (10).

2. The self-drilling bone screw according to claim 1, **characterized in that** the bone screw (10) has a length of 3 to 7 mm.

3. The self-drilling bone screw according to one of claims 1 to 2, **characterized in that** a thread core (24) has a diameter of 1.0 to 1.3 mm.

4. The self-drilling bone screw according to claim 3, **characterized in that** the thread core (24) has a diameter of 1.05 to 1.2 mm.

5. The self-drilling bone screw according to one of claims 1 to 4, **characterized in that** the thread core (24) has a conical shape.

6. The self-drilling bone screw according to one of claims 1 to 5, **characterized in that** the bone screw (10) comprises a head (12) with a torque receiving structure (26), the torque receiving structure (26) comprising square-shaped edges (28).

7. The self-drilling bone screw according to claim 6, **characterized in that** the head (12) of the bone screw (10) has a diameter of 2.0 to 3.0 mm.

8. An implant system comprising a self-drilling bone screw (10) according to one of claims 1 to 7 and a plate (30), wherein the bone screw (10) is adapted to penetrate a bore (32) constructed in the plate (30), wherein a lower face of a head (12) of the bone screw (10) is adapted to a recess (34) constructed in a surface of the plate (30).

9. The implant system according to claim 8, **characterized in that** the lower face of the head (12) of the bone screw (10) has a conical shape.

## Revendications

1. Vis d'ostéosynthèse auto-forante (10) présentant une tige (16) munie d'un filetage (14) ainsi qu'une zone pointue formée sans flancs, **caractérisée en ce que** le filetage (14) présente un pas de filet compris entre 0,8 et 0,9 mm, la vise d'ostéosynthèse (10) possède un angle au sommet compris entre 34 et 43° et le début d'un flanc de filet (20) d'un premier pas de vis (22) faisant suite à la zone pointue de la vis d'ostéosynthèse (10) est disposé à une distance comprise entre 0,2 et 0,5 mm d'une pointe (18) de la vis d'ostéosynthèse (10).

2. Vis d'ostéosynthèse auto-forante selon la revendication 1, **caractérisée en ce que** la vis d'ostéosynthèse (10) présente une longueur comprise entre 3 et 7 mm.

3. Vis d'ostéosynthèse auto-forante selon l'une des revendications 1 à 2, **caractérisée en ce qu'**un corps de filet (24) présente un diamètre compris entre 1,0 et 1,3 mm.

4. Vis d'ostéosynthèse auto-forante selon la revendication 3, **caractérisée en ce que** le corps de filet (24) présente un diamètre compris entre 1,05 et 1,2 mm.

5. Vis d'ostéosynthèse auto-forante selon l'une des revendications 1 à 4, **caractérisée en ce que** le corps de filet (24) se présente sous forme conique.

6. Vis d'ostéosynthèse auto-forante selon l'une des revendications 1 à 5, **caractérisée en ce que** la vis d'ostéosynthèse (10) comporte une tête (12) munie d'une structure réceptrice de couple de rotation (26), ladite structure réceptrice de couple de rotation (26) présentant des bords (28) de forme anguleuse.

7. Vis d'ostéosynthèse auto-forante selon la revendication 6, **caractérisée en ce que** la tête (12) de la vis d'ostéosynthèse (10) présente un diamètre compris entre 2,0 et 3,0 mm.

8. Système d'implant comportant une vis d'ostéosynthèse auto-forante (10) selon l'une des revendications 1 à 7 et une plaque (30), la vis d'ostéosynthèse (10) étant aménagée pour pénétrer dans un alésage (32) formé dans la plaque (30), une face inférieure d'une tête (12) de la vis d'ostéosynthèse (10) étant adaptée à une noyure (34) formée dans une surface de la plaque (30).

9. Système d'implant selon la revendication 8, **caractérisé en ce que** la face inférieure de la tête (12) de la vis d'ostéosynthèse (10) se présente sous forme conique.
